# EUROPÄISCHE PATENTSCHRIFT

(11) **EP 0 628 805 B1**
(45) Veröffentlichungstag und Bekanntmachung des Hinweises auf die Patenterteilung: **18.09.1996**
(21) Anmeldenummer: 94890069.1
(22) Anmeldetag: 18.04.1994
(51) Int. Cl.: G01N 21/64

(54) **Lumineszenzoptischer Indikator zur Bestimmung der Aktivität von Alkalimetallionen in einer Probenlösung**
Luminescent optical indicator for measuring the activity of alkali metal ions in a sample solution
Indicateur optique luminescent pour déterminer l'activité des ions alcalins dans une solution d'échantillon

(30) Priorität: 09.06.1993 AT 1129/93
(43) Veröffentlichungstag der Anmeldung: 14.12.1994
(73) Patentinhaber: AVL Medical Instruments AG, CH-8207 Schaffhausen (CH)
(72) Erfinder: Lippitsch, Max, Prof.Dr, A-8044 Graz (AT); Draxler, Sonja, Dr., A-8047 Graz (AT); Leiner, Marco Jean-Pierre, Dr., A-8045 Graz (AT)
(74) Vertreter: Krause, Walter, Dr. Dipl.-Ing.

(56) Entgegenhaltungen:
- EP-A- 0 397 641
- WO-A-92/10739
- DE-A- 3 702 210
- US-A- 4 762 799
- 'FIBER OPTIC CHEMICAL SENSORS AND BIOSENSORS, VOLUME I' , CRC PRESS , BOCA RATON ANN ARBOR BOSTON LONDON EDITOR WOLFBEIS * Seite 336, Absatz 4 - Seite 341, Absatz 3 *

## Beschreibung

Die Erfindung betrifft einen lumineszenzoptischen Indikator zur Bestimmung der Aktivität von Alkalimetallionen in einer Probenlösung, wobei die Probenlösung den Indikator zumindest indirekt kontaktiert, sowie eine Sensoranordnung mit einem derartigen Indikator.

Die Messung der Aktivität von Alkalimetallionen hat insbesondere medizinische Bedeutung. So wird das Elektrolytgleichgewicht im Blut durch Natrium und Kalium wesentlich mitbestimmt, und Lithium spielt eine wesentliche Rolle für die Gehirnfunktion.

Für die Messung von Ionenkonzentrationen bzw. Aktivitäten sind eine Reihe unterschiedlicher Methoden bekannt, wie z.B. Potentiometrie und optische Messungen mit Hilfe sogenannter Optoden.

Potentiometrische Elektroden werden seit vielen Jahren zur Bestimmung von Kationen in Probelösungen verwendet. Bei der Durchführung der Bestimmung kommt die ionenselektive Membran der Elektrode mit der flüssigen Probe in Kontakt. An der Membranoberfläche reagiert der in der Membran enthaltene ionenselektive Ionophor mit dem zu bestimmenden Ion in der Probelösung. Hierbei wird ein Membranpotential erzeugt, dessen Größe von der Konzentration des in der Probelösung vorliegenden Ions abhängt. Obwohl das Ausmaß der Komplexbildung sehr gering ist, können potentiometrische Methoden die resultierenden Potentiale verlässlich messen.

Entscheidende Nachteile ionenselektiver Elektroden sind
- die Notwendigkeit eines Referenzelementes,
- mangelnde Miniaturisierbarkeit
- Empfindlichkeit gegen elektrochemische Potentiale und elektromagnetische Störungen
- die Notwendigkeit einer individuellen, der Messung vorgelagerten Kalibration.

Optoden hingegen benötigen kein Referenzelement. Die optischen Signale sind unabhängig von externen Potentialen und Stromflüssen. Bisher bekannt gewordene Optoden zur Messung von Ionenkonzentrationen beruhen auf der Messung der von der Ionenaktivität abhängigen Fluoreszenzintensität eines Fluorophors bzw. der Lichtabsorption eines Chromophors. Bedingt durch die Beeinflussung der optischen Signale durch die Indikatorkonzentration, die Intensität der verwendeten Lichtquelle und die Variabilität der Detektorempfindlichkeit ist allerdings eine Kalibrierung vor Meßbeginn mit einem geeigneten Kalibriermedium erforderlich.

Optoden ermöglichen die Entwicklung miniaturisierter Meßelemente. Dies ist von besonderer Bedeutung im Fall sehr kleiner Probenvolumina, und für das Design von Kathedern zur invasiven Messung in der klinischen Chemie und in der Medizin.

In neuerer Zeit wurden verschiedene optische Sensoren zur Messung von Alkalimetallionenkonzentrationen angegeben, die bevorzugt auf einer, durch die Ionen hervorgerufenen Änderung der Lichtabsorption bzw. der Fluoreszenzintensität eines geeigneten Indikators beruhen (oder allgemeiner, der Intensität der Photolumineszenz; im folgenden wird der Einfachheit halber der Begriff Fluoreszenz gebraucht, der aber alle Arten von Photolumineszenz umfassen soll).

Aus der AT-B 384 677 ist ein optischer Kaliumsensor bekannt, welcher den kaliumselektiven Ionophor Valinomycin und einen potentialsensitiven Fluorophor in einer dünnen polymeren Membran beinhaltet. Ähnlich wie bei den eingangs erwähnten Elektroden, führt die Aufnahme von Kaliumionen durch den Ionophor zu einer Potentialdifferenz zwischen der Trennfläche der Membran und der Probenlösung. Das, von der Kaliumkonzentration abhängige elektrische Potential ändert die Fluoreszenzintensität des potentialsensitiven Fluorophors. Allerdings ist bei derartigen Anordnungen das Ausmaß der Komplexierung von Kalium durch den Ionophor sehr gering. Hierdurch ist die Sensitivität solcher Anordnungen klein und damit eine präzise quantitative Bestimmung der Kationenkonzentration der Probe nicht erreichbar. Aus der EP-A 0 358 991 ist bekannt, daß die erwähnten Nachteile dadurch beseitigt werden, wenn dem den kationenselektiven Ionophor enthaltenden Trägermaterial ein Chromophor zugesetzt wird, der ein anderes Kation in die Probenlösung abgeben kann, wenn die zu bestimmenden Kationen der Probe vom kationenselektiven Ionophor in der Membran komplexiert werden. Das Ausmaß der Komplexierung und die Änderung des optischen Signals ist dadurch wesentlich grösser als bei der Anordnung nach der AT-B 384 677. Es ist jedoch bekannt, daß das Ausmaß der Änderungen des optischen Signals nicht nur von der Aktivität des zu bestimmenden Kations in der Probelösung abhängt, sondern auch von der Aktivität des von der Trägermembran in die Probelösung abzugebenden Kations. Aus der Literatur (H. He und O.S. Wolfbeis; SPIE Vol. 1368 [1990]) ist ferner bekannt, daß der Chromophor gemäß EP-A 0 358 991 auch durch einen Fluorophor ersetzt werden kann. In der Praxis, wo das abzugebende Kation meist ein Proton ist, bedeutet dies, daß die Messung von Alkalimetallionen mittels solcher Anordnungen auch vom pH-Wert der Probe abhängt.

Sousa und Larson (Journal of the American Chemical Society 99, 307 [1977]), beschreiben die Änderung der Intensität der intrinsischen Fluoreszenz von Kronenethern durch Komplexierung mit Alkalimetallionen.

De Silva und Mitarbeiter beschreiben (z.B. Journal of the Chemical Society Perkin Transactions II 1989, 1559-1564 [1989]) Ionensensoren, bei denen die Bindung von Ionen einen Elektronentransfer zwischen einem Ionophor und einem Fluorophor unterdrückt und damit die Fluoreszenzintensität erhöht. Aoki et al. (Journal of the Chemical Society Chemical Communications 1992, 730-732 [1992]) demonstrieren, daß in einem System bestehend aus Pyren, Calixaren und Nitrobenzol durch Ionenbindung die Fluoreszenzintensität erhöht wird. He et al. (Analytical Chemistry 65, 123-127 [1993]) beschreiben einen Sensor, bei dem die Fluoreszenzintensität eines ionenunabhängigen Fluorophors durch die ionenabhängige Absorption eines Ionophors verändert wird.

Die US-PS 5 154 890 beschreibt ein ähnliches Prinzip. Hierbei ist ein kaliumselektiver Fluoroionophor immobilisiert in einem ionenpermeablen polymeren Gel, dessen Fluoreszenzintensität selektiv von der Kaliumkonzentration der Probe abhängig ist.

Alle genannten Vorrichtungen basieren auf der Fluoreszenzintensität als Informationsträger. Derartige Vorrichtungen haben jedoch den entscheidenden Nachteil, daß das Meßsignal durch unvermeidliche Änderungen der Intensität der Lichtquelle, des Lichtdurchsatzes durch das optische System, der Empfindlichkeit des Detektors und insbesondere der Konzentration des Indikators in schwer korrigierbarer Weise beeinflußt wird, was ihre praktische Anwendbarkeit beeinträchtigt. Dieser Umstand macht es erforderlich, daß in der Produktion jedes einzelne Sensorelement individuell kalibriert werden muß und vor einem Meßeinsatz (bzw. bei längerer Meßdauer periodisch wiederkehrend während des Einsatzes) eine Nachkalibrierung vorgenommen werden muß.

Die genannten Nachteile könnten an sich dadurch vermieden werden, daß anstelle der Fluoreszenzintensität die Fluoreszenzabklingzeit als Meßgröße herangezogen wird. Die Fluoreszenzabklingzeit ist eine inhärente Eigenschaft des verwendeten Indikators und seiner molekularen Umgebung. Sie hängt nicht von der Dicke der Sensorschicht, der Konzentration der Indikatorsubstanz, der Intensität der Lichtquelle, der Empfindlichkeit des Detektors und den optischen Eigenschaften des Meßsystems ab. Herstellungsbedingte Unterschiede zwischen einzelnen Sensorelementen führen daher nicht zu unterschiedlichen Kennlinien, sodaß eine an einem Element bestimmte Kalibrierfunktion auch für alle übrigen Elemente Gültigkeit hat, wodurch eine individuelle Kalibrierung entfällt. Ebenso führen allfällige alterungsbedingte Änderungen eines Sensorelementes nicht zu einer Änderung der Kennlinie, was eine der Messung vorangehende Nachkalibrierung unnötig macht.

Von Bacon und Demas (US-PS 5 030 420) sowie Lippitsch et al. (Analytica Chimica Acta 205, 1-6 [1988]) wurde ein derartiger Abklingzeitsensor für die Messung von Sauerstoff vorgeschlagen. Abklingzeitsensoren für pH (WO 92/10739), Calcium (Lakowicz et al., Cell Calcium 13, 131-147 [1992]), Aluminium, Gallium und Indium (Caroll et al., Analytical Chemistry 61, 1768-1772 [1989]) wurden bisher beschrieben. Abklingzeitsensoren für Alkalimetallionen wurden bisher nicht bekannt.

Aus der AT-PS 393 035 ist weiters ein Verfahren zur quantitativen Bestimmung eines chemischen Parameters einer Probe bekannt, bei welchem der Indikator aus zwei in räumlich engem Kontakt stehenden Substanzen, nämlich einem Fluorophor und einem Chromophor besteht. Während der Fluorophor auf den zu bestimmenden Parameter nicht reagiert, erfährt der Chromophor eine Änderung seines Absorptionsspektrums. Da sich das Emissionsspektrum des Fluorophors teilweise mit dem Absorptionsspektrum des Chromophors überlappt, kommt es zwischen den beiden Substanzen zu einem Energietransfer, wodurch sich die Fluoreszenzabklingzeit des Fluorophors in Abhängigkeit des chemischen Parameters verringert.

Aufgabe der Erfindung ist es, lumineszenzoptische Indikatoren auf der Basis der Abklingzeitmessung für die Bestimmung der Aktivität von Alkalimetallionen sowie Sensoranordnungen mit derartigen Indikatoren anzugeben.

Erfindungsgemäß wird dies dadurch gelöst, daß der Indikator zumindest drei chemisch aneinander gebundene Komponenten aufweist, nämlich einen Fluorophor, eine funktionelle Gruppe, mit welcher die Lumineszenzabklingzeit des Fluorophors beeinflußbar ist, und einen Ionophor, welcher selektiv und reversibel die zu messenden Alkalimetallionen bindet, wodurch die Wirkung der funktionellen Gruppe auf die Abklingzeit des Fluorophors in Abhängigkeit der Aktivität der Alkalimetallionen veränderbar ist.

Weiters ist vorgesehen, daß das Absorptionsmaximum des Fluorophors im Wellenlängenbereich zwischen 400 und 1200 nm liegt, sowie daß die Lumineszenzabklingzeit des Fluorophors größer 10 ns ist.

Zusammenfassend besteht ein erfindungsgemäßer Indikator somit aus:
- mindestens einem Fluorophor, mit einer Absorption bevorzugt im sichtbaren oder nahen infraroten Spektralbereich (400-1200 nm) und einer bevorzugten Fluoreszenzabklingzeit > 10 ns,
- mindestens einer funktionellen Gruppe, die durch Wechselwirkung mit dem Fluorophor dessen Fluoreszenzabklingzeit beeinflußt,
- mindestens einem Ionophor, der spezifisch und reversibel eine bestimmte Art von Alkalimetallionen bindet, durch diese Bindung die Wechselwirkung der funktionellen Gruppe mit dem Fluorophor verändert und auf diese Weise die Fluoreszenzabklingzeit des Fluorophors zu einer Funktion der Konzentration des betreffenden Alkalimetallions macht.

Die drei Komponenten befinden sich in unmittelbarer räumlicher Nähe zueinander, und zwar entweder dadurch, daß sie direkt aneinander chemisch gebunden sind oder auch in einer geeigneten Matrix an unmittelbar beieinanderliegenden Orten immobilisiert sind.

Im folgenden werden einige Beispiele für verwendbare Fluorophore, funktionelle Gruppen und Ionophore mit ihren für die Erfindung wesentlichen Eigenschaften genannt.

### Verwendete Abkürzungen:

- lₐ: langwelliges Ende der Absorptionsbande
- t_{f}: Fluoreszenzabklingzeit
- K_{S}: Bindungskonstante Ionophor/Kation
- pKs: Dekadischer Logarithmus von K_{S}

### Beispiele für Fluorophore:

| Substanz | lₐ (nm) | t_{f}(ns) |
|---|---|---|
| 2-Aminoanthracen | 480 | 31 |
| Rubrene | 560 | 17 |
| Decacyclen | 490 | 28 |
| Ruthenium-Bipyridyl | 550 | 545 |
| Ruthenium-Phenanthrolin | 447 | 950 |

Weiters sind poly- und heterozyklische Aromaten und Metallionenkomplexe mit heterozyklischen Liganden als Fluorophore denkbar, sofern sie durch funktionelle Gruppen in ihrer Fluoreszenzabklingzeit beeinflußbar sind.

### Beispiele für funktionelle Gruppen:

| Substanz | Art der t-Beeinflussung |
|---|---|
| Nitro-, Cyano- | Elektronenakzeptor |
| Amino-, Methoxy- | Elektronendonor |
| Viologen | Elektronenakzeptor |
| Halogenide u. Pseudohalogenide | Elektronenakzeptor |

### Beispiele für Ionophore:

| Substanz | Kation | pKS |
|---|---|---|
| Calix[4] arenester | Na⁺ | 4.3 |
| | K⁺ | 2.9 |
| 1,4-Benzodioxin-23-crown-7-lariatether | K⁺ | 3.0 |
| | Na⁺ | <0.6 |
| 2,9-Dibutyl-1,10-phenanthrolin | Li⁺ | ca.4.5 |
| | Na⁺ | <2 |

Im folgenden werden erfindungsgemäße Indikatoren zur Bestimmung der Na⁺-, K⁺- und Li⁺-Aktivität einer Probe beschrieben. Diese Beispiele dienen lediglich der Erläuterung der Erfindung, sie sollen jedoch den Erfindungsgegenstand in keiner Weise einschränken.
1. Indikator zur Messung der Aktivität von Na+ Ionen 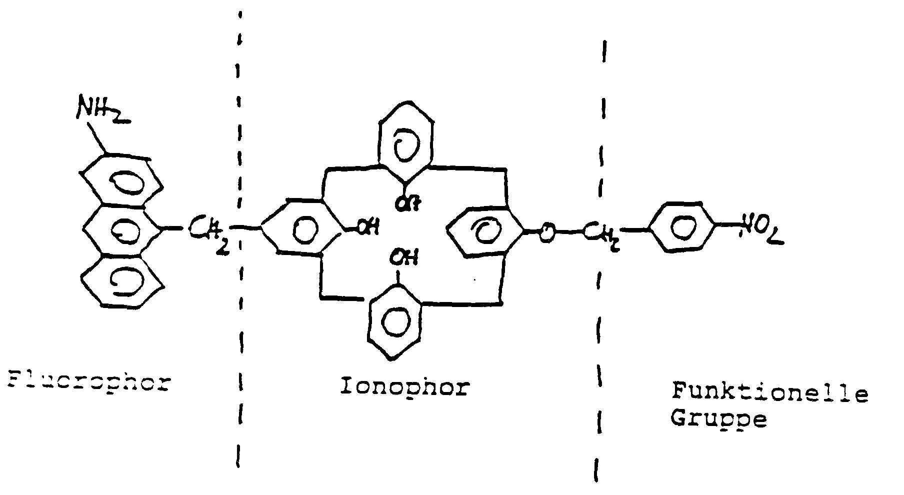
   Der Fluorophor ist in diesem Beispiel 2-Aminoanthracen, die funktionelle Gruppe Nitrophenylmethyl, der Ionophor ein Calix[4]aren. Hat der Ionophor kein Kation gebunden, so sind die Ringe, an denen die funktionelle Gruppe bzw. der Fluorophor gebunden sind, frei drehbar. Durch die thermische Bewegung können sich diese beiden Komponenten somit häufig einander nähern, wobei die Nitrogruppe fluoreszenzlöschend auf den Fluorophor wirkt, die Fluoreszenzabklingzeit wird verkürzt. Der Ionophor hat eine hohe Spezifität für die Bindung von Na⁺. Wird ein derartiges Ion gebunden, so wird die Position der Ringe durch Wechselwirkung der Phenolsauerstoffe mit dem Ion fixiert, eine Löschung kann nicht mehr stattfinden und die Abklingzeit geht annähernd auf den Wert des ungequenchten Fluorophors.
2. Indikatoren zur Messung der Aktivität von K+ Ionen 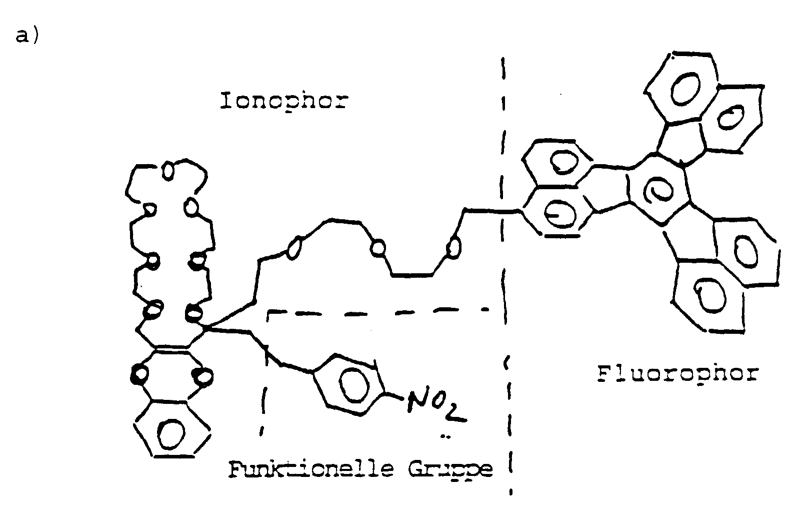 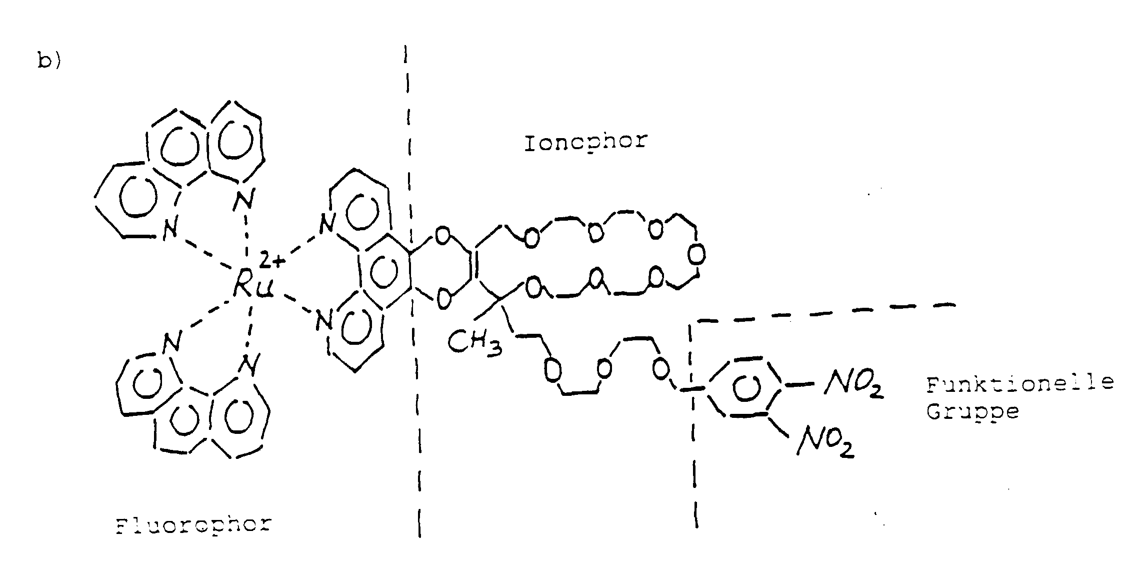
   Der Fluorophor ist in Beispiel a) Decacyclen, die funktionele Gruppe Nitrophenylmethyl, der Ionophor ein Benzodioxin-23-crown-7-lariatether. In Beispiel b) wird als Fluorophor tri-Ruthenium-Phenanthrolin, als funktionelle Gruppe Dinitrobenzo und als Ionophor ein Benzodioxin-23-crown-7-lariatether verwendet. Hat der Ionophor kein Kation gebunden, so ist der Sei tenarm, an dem die funktionelle Gruppe gebunden ist, frei beweglich. Durch die thermische Bewegung wird diese somit häufig nahe an den Fluorophor kommen, wodurch dessen Fluoreszenz gelöscht und seine Fluoreszenzabklingzeit verkürzt wird. Der Ionophor hat eine hohe spezifität für die Bindung von K⁺. Wird ein derartiges Ion gebunden, so wird die Position des Seitenarms durch Wechselwirkung der Estersauerstoffe mit dem Ion fixiert, eine Löschung kann nicht mehr stattfinden und die Abklingzeit geht annähernd auf den Wert des ungequenchten Fluorophors.
3. Indikator zur Messung der Aktivität von Li+ Ionen 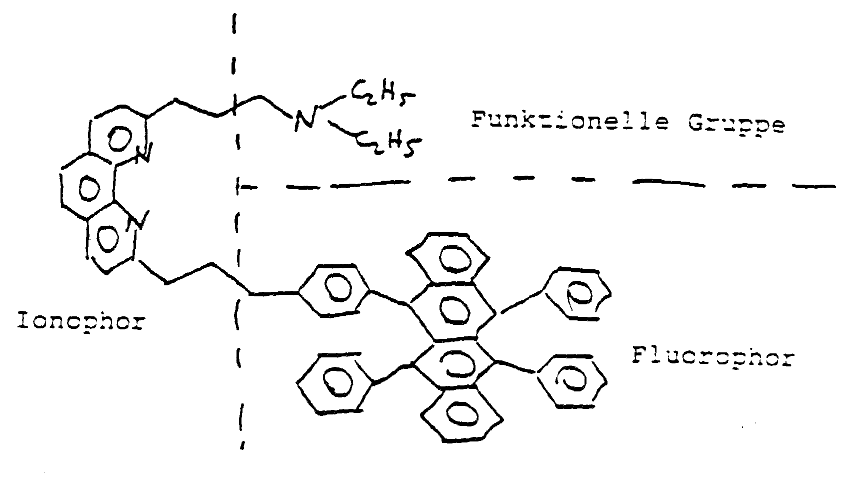
   Der Fluorophor ist in diesem Beispiel Rubren, die funktionelle Gruppe Diethylamin, der Ionophor Dibutylphenanthrolin. Hat der Ionophor kein Kation gebunden, so sind die Butylreste, an denen die funktionellen Gruppen gebunden sind, frei beweglich. Durch die thermische Bewegung werden diese beiden Komponenten somit häufig nahe an den Fluorophor gebracht werden, wobei die Aminogruppe fluoreszenzlöschend ist und die Fluoreszenzabklingzeit verkürzt wird. Der Ionophor hat eine hohe Spezifität für die Bindung von Li⁺. Wird ein derartiges Ion gebunden, so werden die Aminogruppen durch Wechselwirkung mit dem Ion fixiert und die positive Ladung des Ions bindet die freien Ionenpaare der Stickstoffatome. Eine Löschung kann daher nicht mehr stattfinden und die Abklingzeit geht annähernd auf den Wert des ungequenchten Fluorophors.

In einer Sensoranordnung mit dem beschriebenen, erfindungsgemäßen Indikator ist vorgesehen, daß der Indikator in einer hydrophilen, ionenpermeablen Matrix auf einem für die Anregungs- und Meßstrahlung durchlässigen Träger vorliegt, wobei der Indikator in der hydrophilen, ionenpermeablen Matrix vorzugsweise physikalisch gelöst vorliegt. Es ist jedoch auch möglich, den Indikator elektrostatisch oder kovalent in der Matrix zu binden.

Das Anbringen der alkalimetallionenselektiven Fluoreszenzindikatoren in eine ionenpermeablen hydrophilen Polymermatrix erfolgt besonders kostengünstig durch physikalisches Lösen der Indikatoren in der polymeren Trägermatrix. Da, im Gegensatz zur Messung der Fluoreszenzintensität oder der Lichtabsorption, die Messung der Fluoreszenzabklingzeit unabhängig von Veränderungen der Konzentration des Indikators und von geometrischen Veränderungen des Lichtweges ist (hervorgerufen durch von physikalisch-chemischen Eigenschaften des Meßgutes abhängigem Quellen der polymeren Matrix), ist die Meßanordnung unempfindlich gegen schwache Ausbleicheffekte, Ausdiffundieren des Indikators in die Probe, sowie geometrische Veränderungen des Lichtweges. Diese Variante der Immobilisierung ist besonders vorteilhaft, wenn die Messanordnung als Einwegmeßelement verwendet wird.

Es ist auch möglich, die erfindungsgemäßen Indikatoren durch chemische Immobilisierung kovalent an die Polymermatrix zu binden. Dies ist dann besonders vorteilhaft, wenn die Meßanordnung längere Zeit mit der Probe in Kontakt steht (kontinuierliche Messungen, Langzeitmessungen) oder, verhindert werden soll, daß die Probe mit der Indikatorsubstanz kontaminiert werden kann (in-vivo Messungen). Zur chemischen Immobilisierung der Indikatoren können eine Reihe von in der Literatur bereits beschriebenen Methoden angewendet werden, wie z.B. von E. Koller und O.S. Wolfbeis in "Fiber Optic Chemical Sensors and Biosensors", Vol. 1, chapter 7, O.S. Wolfbeis (ed.), CRC Press, Boca Raton 1991 beschrieben.

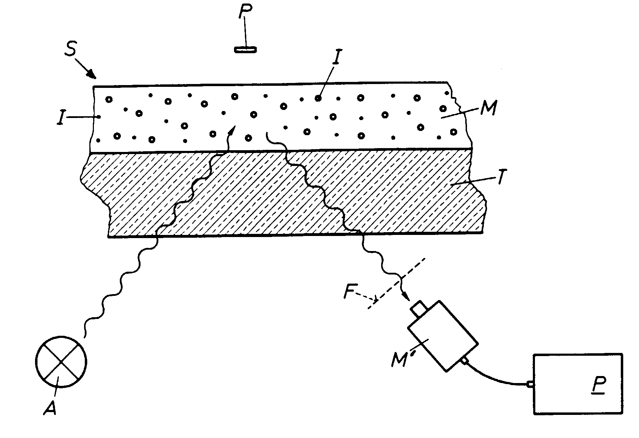

Aus der einzigen Abb. ist eine typische Sensoranordnung mit dem erfindungsgemäßen Indikator ersichtlich. Der lumineszenzoptische Indikator I zur Bestimmung der Aktivität von Alkalimetallionen (z.B. Na⁺-Ionen) in einer Probenlösung P liegt in einer hydrophilen, ionenpermeablen Matrix M vor und ist dort vorzugsweise physikalisch gelöst. Die ionenpermeable Matrix M bildet eine Schichte auf einem für die Anregungs- und Meßstrahlung durchlässigen Träger T. Weiters ist die mit A bzw. M' gekennzeichnete Anregungs- und Meßeinrichtung ersichtlich, welche mit dem Indikator I in optischem Kontakt stehen. Die Signale der Meßeinrichtung M' werden zur Erfassung der Abklingzeit bzw. deren Änderung und der damit zusammenhängenden Aktivitätsbestimmung einer Auswerteeinheit P zugeführt.

Es ist auch möglich, den aus Träger T, Matrix M und darin gelösten Indikator I bestehenden Sensor S bis zur (Einmal-) Messung trocken zu lagern wobei die polymere, hydrophile Matrix M erst durch die wäßrige Probe P zum Quellen gebracht wird. Hydrophyle polymere Trägermaterialien, welche in Kontakt mit wäßrigen Proben innerhalb weniger Sekunden quellen, sind bekannt. Ein wesentlicher erfindungsgemäßer Vorteil ist, daß eine der Messung vorgelagerte Kalibration der Meßanordnung mit Hilfe einer Kalibrierflüssigkeit nicht erforderlich ist. Im Gegensatz zu den konventionellen optischen Meßanordnungen für Alkalimetallionen, ist das Meßsignal unabhängig von Schwankungen der Intensität der Lichtquelle, der Detektorempfindlichkeit und der Indikatorkonzentration. Die von der Meßgröße abhängige Kennlinie der Meßanordnung ist nur abhängig von der Art des Indikators und dem verwendeten polymeren Trägermaterial.

Als ionenpermeable polymere Matrix M eignen sich besonders Hydrogele, Polyvinylalkohole, Polyglycole, Polysaccharide, Polyacrylate, Polyacrylamide, Polyimine, Polyurethane und deren Derivate. Beispiele für den Träger T der Matrix M sind Lichtleiter, Glasplatten oder Kunststoffolien wie z.B. Polyesterfolien, Polycarbonatfolien o.ä.

Die Messung der Fluoreszenzabklingzeit kann, wie an sich bekannt, in zwei grundsätzlich unterschiedlichen Arten vorgenommen werden, nämlich einerseits durch Anregung mit kurzen Lichtimpulsen und zeitaufgelöste Registrierung der nachfolgenden Fluoreszenz, oder andererseits durch Anregung mit hochfrequent moduliertem Licht und Messung der Phasenverschiebung zwischen der Modulation des Anregungs- und des Fluoreszenzlichtes bzw. der Modulationstiefe desselben. Beide Methoden können für die erfindungsgemäße Sensoranordnung Anwendung finden.

In einem Ausführungsbeispiel wird der Indikator I, z. B. in einer Polymermatrix, immobilisiert und mit der Probenlösung P in Kontakt gebracht. Die Anregung der Fluoreszenz erfolgt mit einer Kurzzeitlichtquelle, z. B. einer koaxialen Blitzlampe, einem gepulsten Laser oder einer gepulsten Leuchtdiode. Die Fluoreszenz wird durch ein Kantenfilter F vom Anregungslicht getrennt und mit einem schnellen Photodetektor (Photomultiplier, pin-Photodiode, Avalanchphotodiode) detektiert. Die Abklingzeit wird durch geeignete elektronische Maßnahmen gemessen. Über eine einmal abgespeicherte Eichfunktion können die gemessenen Abklingzeiten auf Ionenkonzentrationen bzw. Aktivitäten umgerechnet werden.

Bei Verwendung der Phasenfluorometrie wird die Lichtquelle (Lampe, Laser, Leuchtdiode) mit hoher Frequenz moduliert, die Fluoreszenz spektral abgetrennt und mit einem schnellen Photodetektor registriert. Die Phasenverschiebung zwischen der Modulation des Anregungs- und der des Fluoreszenzlichtes ist dann eine Funktion der Fluoreszenzabklingzeit und somit der Ionenkonzentration.

## Patentansprüche

1. Lumineszenzoptischer Indikator zur Bestimmung der Aktivität von Alkalimetallionen in einer Probenlösung, wobei die Probenlösung den Indikator zumindest indirekt kontaktiert, **dadurch gekennzeichnet**, daß der Indikator zumindest drei chemisch aneinander gebundene Komponenten aufweist, nämlich einen Fluorophor, eine funktionelle Gruppe, mit welcher die Lumineszenzabklingzeit des Fluorophors beeinflußbar ist, und einen Ionophor, welcher selektiv und reversibel die zu messenden Alkalimetallionen bindet, wodurch die Wirkung der funktionellen Gruppe auf die Abklingzeit des Fluorophors in Abhängigkeit der Aktivität der Alkalimetallionen veränderbar ist.

2. Indikator nach Anspruch 1, **dadurch gekennzeichnet,** daß das Absorptionsmaximum des Fluorophors im Wellenlängenbereich zwischen 400 und 1200 nm liegt.

3. Indikator nach Anspruch 1 oder 2, **dadurch gekennzeichnet,** daß die Lumineszenzabklingzeit des Fluorophors größer 10 ns ist.

4. Indikator nach einem der Ansprüche 1 bis 3, **dadurch gekennzeichnet,** daß der Fluorophor ein 2-Aminoanthracen, Rubren, Decacyclen, tris-Ruthenium-Bipyridyl oder tris-Ruthenium-Phenanthrolin ist.

5. Indikator nach einem der Ansprüche 1 bis 4, **dadurch gekennzeichnet,** daß die funktionelle Gruppe eine Nitro-, Cyano-, Amino-, Methoxygruppe, ein Viologen, ein Halogen oder ein Pseudohalogen ist.

6. Indikator nach einem der Ansprüche 1 bis 5, **dadurch gekennzeichnet,** daß der Ionophor ein Calix[4]arenester, 1,4-Benzodioxin-23-crown-7-lariatether, oder 2,9-Dibutyl-1,10-phenanthrolin ist.

7. Indikator nach einem der Ansprüche 1 bis 3, **dadurch gekennzeichnet,** daß der Fluorophor ein durch funktionelle Gruppen in seiner Fluoreszenzabklingzeit beeinflußbarer poly- und heterozyklischer Aromat oder ein Metallionenkomplex mit heterozyklischen Liganden ist.

8. Indikator nach einem der Ansprüche 1 bis 3, **dadurch gekennzeichnet**, daß zur Bestimmung der Aktivität der Na⁺-Ionen einer Probenlösung als Fluorophor 2-Aminoanthracen, als funktionelle Gruppe Nitrophenylmethyl und als Ionophor ein Calix[4]aren vorgesehen ist.

9. Indikator nach einem der Ansprüche 1 bis 3, **dadurch gekennzeichnet**, daß zur Bestimmung der Aktivität der K⁺-Ionen einer Probenlösung als Fluorophor Decacyclen, als funktionelle Gruppe Nitrophenylmethyl und als Ionophor einen Benzodioxin-23-crown-7-lariatether vorgesehen ist.

10. Indikator nach einem der Ansprüche 1 bis 3, **dadurch gekennzeichnet**, daß zur Bestimmung der Aktivität der K⁺-Ionen einer Probenlösung als Fluorophor tris-Ruthenium-Phenanthrolin, als funktionelle Gruppe Dinitrobenzol und als Ionophor einen Benzodioxin-23-crown-7-lariatether vorgesehen ist.

11. Indikator nach einem der Ansprüche 1 bis 3, **dadurch gekennzeichnet**, daß zur Bestimmung der Aktivität der Li⁺-Ionen einer Probenlösung als Fluorophor Rubren als funktionelle Gruppe Diethylamin und als Ionophor Dibutylphenanthrolin vorgesehen ist.

12. Sensoranordnung mit einem lumineszenzoptischen Indikator (I) zur Bestimmung der Aktivität von Alkalimetallionen einer Probenlösung (P), wobei die Probenlösung (P) den Indikator zumindest indirekt kontaktiert und der Indikator mit einer Anregungs- (A) und Meßeinrichtung (M) in optischem Kontakt steht, **dadurch gekennzeichnet**, daß der Indikator zumindest drei chemisch aneinander gebundene Komponenten aufweist, nämlich einen Fluorophor, eine funktionelle Gruppe, mit welcher die Lumineszenzabklingzeit des Fluorophors beeinflußbar ist und einen Ionophor, welcher selektiv und reversibel die zu messenden Alkalimetallionen bindet, wodurch die Wirkung der funktionellen Gruppe auf die Abklingzeit des Fluorophors in Abhängigkeit der Aktivität der Alkalimetallionen veränderbar ist, sowie daß der Indikator (I) in einer hydrophilen, ionenpermeablen Matrix (M) auf einem für die Anregungs- und Meßstrahlung durchlässigen Träger (T) vorliegt.

13. Sensoranordnung nach Anspruch 12, **dadurch gekennzeichnet**, daß der Indikator (I) in der hydrophilen, ionenpermeablen Matrix (M) physikalisch gelöst vorliegt.

14. Sensoranordnung nach Anspruch 12, **dadurch gekennzeichnet**, daß der Indikator (I) in der hydrophilen, ionenpermeablen Matrix (M) elektrostatisch gebunden vorliegt.

15. Sensoranordnung nach Anspruch 12, **dadurch gekennzeichnet**, daß der Indikator (I) in der hydrophilen, ionenpermeablen Matrix (M) kovalent gebunden vorliegt.

## Claims

1. A luminescence-optical indicator for determining the activity of alkali metal ions in a sample solution, the said solution being at least in indirect contact with the indicator, **wherein** the indicator has at least three components that are chemically bound to one another, i.e., a fluorophor, a functional group with which the luminescence decay time of the fluorophor can be influenced, and a ionophor selectively and reversibly binding the alkali metal ions to be measured, such that the effect of the functional group on the decay time of the fluorophor can be altered depending on the activity of the alkali metal ions.

2. An indicator as in claim 1, **wherein** the absorption maximum of the fluorophor is in the wavelength range of 400-1,200 nm.

3. An indicator as in claim 1 or 2, **wherein** the luminescence decay time of the fluorophor is greater than 10 ns.

4. An indicator as in any of claims 1 to 3, **wherein** the fluorophor is a 2-aminoanthracene, rubrene, decacyclene, tris-ruthenium-bipyridyl, or tris-ruthenium-phenantroline.

5. An indicator as in any of claims 1 to 4, **wherein** the functional group is a nitro-, cyano-, amino-, methoxy-group, a viologene, halide, or a pseudohalide.

6. An indicator as in any of claims 1 to 4, **wherein** the ionophor is a calix(4)arene ester, 1,4-benzodioxin-23-crown-7-lariatether, or 2,9-dibutyl-1,10-phenanthroline.

7. An indicator as in any of claims 1 to 3, **wherein** the fluorophor is a polycyclic and heterocyclic aromatic or a metal ion complex with heterocyclic ligands, whose fluorescence decay time can be influenced by functional groups.

8. An indicator as in any of claims 1 to 3, **wherein** for determination of the activity of the Na⁺ ions of a sample solution 2-aminoanthracene is used as fluorophor, nitrophenylmethyl as functional group, and a calix(4)arene as ionophor.

9. An indicator as in any of claims 1 to 3, **wherein** for determination of the activity of the K⁺ ions of a sample solution decacyclene is used as fluorophor, nitrophenylmethyl as functional group, and a benzodioxin-23-crown-7-lariatether as ionophor.

10. An indicator as in any of claims 1 to 3, **wherein** for determination of the activity of the K' ions of a sample solution tris-ruthenium-phenanthroline is used as fluorophor, dinitrobenzene as functional group, and a benzodioxin-23-crown-7-lariatether as ionophor.

11. An indicator as in any of claims 1 to 3, **wherein** for determination of the activity of the Li⁺ ions of a sample solution rubrene is used as fluorophor, diethylamine as functional group, and dibutylphenanthroline as ionophor.

12. A sensor configuration with a luminescence-optical indicator (I) for determining the activity of alkali metal ions in a sample solution (P), the said solution (P) being at least in indirect contact with the indicator (I) and the said indicator (I) being in optical contact with an excitation and measuring unit (A and M', respectively), **wherein** the indicator has at least three components that are chemically bound to one another, i.e., a fluorophor, a functional group with which the luminescence decay time of the fluorophor can be influenced, and a ionophor selectively and reversibly binding the alkali metal ions to be measured, such that the effect of the functional group on the decay time of the fluorophor can be altered depending on the activity of the alkali metal ions, and wherein the indicator (I) is incorporated in a hydrophilic, ion-permeable matrix (M) on a carrier substrate (T) transparent to the excitation and measurement radiation.

13. A sensor configuration as in claim 12, **wherein** the indicator (I) is physically dissolved in the hydrophilic, ion-permeable matrix (M).

14. A sensor configuration as in claim 12, **wherein** the indicator (I) is incorporated in the hydrophilic, ion-permeable matrix (M) by electrostatic bonding.

15. A sensor configuration as in claim 12, **wherein** the indicator (I) is incorporated in the hydrophilic, ion-permeable matrix (M) by covalent bonding.

## Revendications

1. Indicateur de luminiscence optique pour déterminer l'activité des ions alcalino-métalliques dans une solution d'échantillons, étant en contact au moins indirect avec l'indicateur, caractérisé par au minimum trois composantes entreliées chimiquement à savoir un fluorophore, un groupe fonctionnel agissant sur la durée de retour au zéro de la luminiscence du fluorophore, ainsi qu'un ionophore liant d'une manière selective et reversible les ions alcalino-métalliques à mesurer, de façon que l'effet du groupe fonctionnel sur la durée de retour au zéro de la luminiscence du fluorophore peut être varié selon l'activité des ions alcalino-métalliques.

2. Indicateur selon la revendication 1, caractérisé par un maximum d'absorption du fluorophore étant situé dans une gamme de longueurs d'onde de 400 à 1200 nm.

3. Indicateur selon la revendication 1 ou 2, caractérisé par la durée de retour au zéro de la luminiscence du fluorophore étant au delà de 10 ns.

4. Indicateur selon l'une des revendications 1 à 3, carcatérisé en ce que le fluorophore est représenté par un 2-amino-anthracène, rubrène, déca-cyclène, tris-ruthenium-bi-pyridile ou un tris-ruthenium-phénanthroline.

5. Indicateur selon l'une des revendications 1 à 4, caractérisé en ce qu'on se sert d'un nitro-, cyano-, amino- ou methoxy-groupe, d'un viologène, halogène ou pseudo-halogène comme groupe fonctionnel.

6. Indicateur selon l'une des revendications 1 à 5, caractérisé en ce qu'on se sert d'un calixe[4]arène-éther, ou bien d'un 1,4 benzo-dioxine-23-crown-7-lariat-éther ou d'un 2,9 dibutyle-1,10-phénantroline comme ionophore.

7. Indicateur selon l'une des revendications 1 à 3, caractérisé par l'emploi d'un aromatique polycyclique ou hétérocyclique ou bien d'un complexe d'ions metalliques aux ligands hétérocycliques, dont les durées de retour au zéro de la fluorescence sont influençables par des groupes fonctionnels.

8. Indicateur selon l'une des revendications 1 à 3, **caractérisé** en ce que, pour déterminer l'activité des ions Na+ d'une solution d'échantillons, on se sert comme fluorophore d'un 2-amino-anthracène, comme groupe fonctionnel d'un nitro-phenyle-methyle et comme ionophore d'un calix[4]arène.

9. Indicateur selon l'une des revendications 1 à 3, caractérisé en ce que, pour déterminer l'activité des ions K+ d'une solution d'échantillons, on se sert comme fluorophore d'un déca-cyclene, comme groupe fonctionnel d'un nitro-phenyle-methyle et comme ionophore d'un benzo-dioxine-23-crown-7-lariat-éther.

10. Indicateur selon l'une des revendications 1 à 3, caractérisé en ce que, pour déterminer l'activité des ions K+ d'une solution d'échantillons on se sert d'un tris-ruthenium-phénantroline comme fluorophore, d'un di-nitro-benzène comme groupe fonctionnel et d'un benzo-dioxine-23-crown-lariat-éther comme ionophore.

11. Indicateur selon l'une des revendications 1 à 3, caractérisé en ce qu'il est prévu de déterminer l'activité des ions Li+ d'une solution d'échantillons à l'aide de rubrène figurant comme fluorophore, à l'aide de di-éthyle-amine représentant le groupe fonctionnel et à l'aide de di-butyle-phénantroline servant de ionopnore.

12. Arrangement de capteur à indicateur de luminiscence optique (I) pour déterminer l'activité des ions alcalino-métalliques d'une solution d'échantillons (P), étant en contact au moins indirect avec l'indicateur, et ce dernier à son tour étant en contact optique avec un dispositif d'excitation (A) et un mesureur (M), caractérisé en ce que l'indicateur a au moins trois composantes chimiquement entreliées, à savoir un fluorophore, un groupe fonctionnel susceptible d'influencer la durée de retour au zéro de la luminiscence du fluorophore et un ionophore liant d'une manière selective et reversible les ions alcalino-métalliques à mesurer, de sorte que l'effet du groupe fonctionnel sur la durée de retour au zéro de la luminiscence du fluorophore varie selon l'activité des ions alcalino-métalliques, et que l'indicateur (I) se trouve dans une matrice (M) hydrophile et perméable aux ions, celle-ci étant fixée sur un support (T) perméable au rayonnement d'excitation et de mesurage.

13. Arrangement de capteur selon la revendication 12, caractérisé en ce que l'indicateur (I) est physiquement dissolu dans la matrice (M), qui est hydrophile et perméable aux ions.

14. Arrangement de capteur selon la revendication 12, caractérisé en ce que l'indicateur (I) se trouve lié par électrostatique dans une matrice (M) hydrophile et perméable aux ions.

15. Arrangement de capteur selon la revendication 12, caractérisé en ce que l'indicateur (I) se trouve lié par co-valence dans la matrice (M), qui est hydrophile et perméable aux ions.
